(19) Europäisches Patentamt / European Patent Office / Office européen des brevets

(11) **EP 2 990 797 A1**

(12) **EUROPEAN PATENT APPLICATION**

(43) Date of publication:
**02.03.2016 Bulletin 2016/09**

(51) Int Cl.:
***G01N 33/50*** *(2006.01)*

(21) Application number: **14182508.3**

(22) Date of filing: **27.08.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Rheinisch-Westfälische Technische Hochschule Aachen (RWTH) 52062 Aachen (DE)**

(72) Inventors:
• **Conrath, Uwe 4720 Kelmis (BE)**

• **Büchs, Jochen 52064 Aachen (DE)**
• **Schilling, Jana 52074 Aachen (DE)**
• **Schillheim, Britta 52134 Herzogenrath (DE)**

(74) Representative: **Lasar, Andrea Gisela Maiwald Patentanwalts GmbH Elisenhof Elisenstraße 3 80335 München (DE)**

(54) **Method for identifying substances which prime a defense response**

(57) The present invention relates to a method for identifying substances which prime cells for a stress response by determining the respiration activity of the cells treated with a candidate substance in comparison to cells not treated with the candidate substance.

**EP 2 990 797 A1**

**Description**

**FIELD OF THE INVENTION**

The present invention relates to a method for identifying substances which prime cells for a stress response by determining the respiration activity of the cells treated with a candidate substance in comparison to cells not treated with the candidate substance.

**BACKGROUND OF THE INVENTION**

Plant diseases which are caused by various pathogens such as viruses, bacteria, oomycetes and fungi or abiotic stress such as drought, cold, freeze and salt may lead to significant crop losses of cultivated plants, resulting in economic detriments and in threatening food and feed supply.

Since the last century, chemical pesticides have been used for controlling plant diseases. Nevertheless, at present at least 40% of the possible plant yield is lost due to diseases and abiotic stress such as drought (Oerke et al. (1994) Crop production and crop protection, Elsevier Science B.V, ISBN 0 444 82095 7). Hence, there is still a need for improved methods for controlling plant diseases and abiotic stresses. Due to the low acceptance of genetically modified plants in Europe and because of the increasing demand for substances which are uncritical for the environment, there is a huge interest in using natural or near-natural substances for an effective plant protection.

Natural or near-natural substances are particularly effective, if they stimulate certain parts of the plant's endogenous immune system. This is particularly true for agents which act via the so-called "defense priming" (reviewed in Conrath (2011) Trends in Plant Science 16(10): 524-531; Conrath et al. (2006) Mol. Plant. Microbe Interact. 19(10): 1062-1071). Defense priming prepares the plant's endogenous immune system for a future challenge such as pathogen infection or abiotic stress, but does not directly activate immunity. However, primed plants respond to very low levels of a stimulus, such as biotic or abiotic stress, in a more rapid and robust manner than non-primed plants. Hence, substances which induce defense priming confer an increased stress resistance to plants without reducing the yield. Consequently, there is a strong demand for substances which induce defense priming in plants.

It has been shown that defense priming is not restricted to plant cells, but also occurs in animal cells (see, e.g., Hayes et al. (1991) J. Leukocyte Biol. 50: 176-181). For example, interferon-y or GM-CSF can prime the induction of the expression of the cytokines interferon-$\alpha$, interferon-$\beta$, tumour necrosis factor $\alpha$ and IL-12 which cytokines are involved in the overall defense response (Hayes et al. (1995) Blood 86: 646-650).

However, since the defense priming process does not lead to a direct activation of the defense mechanisms, the search for substances which act via defense priming is rather difficult. In parsley cells, it is possible to identify defense priming substances by measuring the fluorescence induced by these substances (Siegrist et al. (1998) Physiol. Mol. Plant Pathol. 53(4): 223-238). However, this system cannot be used with other, economically more important, plant species or animal cells. Further, this system does not allow the continuous monitoring of the defense response, as the fluorescence is measured only at one specific timepoint.

Consequently, there is still a need for a generally applicable method which enables the identification of substances which prime cells or organisms for a defense response.

**OBJECT AND SUMMARY OF THE INVENTION**

It is, thus, an object of the present invention to provide a method for identifying substances which prime cells or organisms for a stress response.

This and further objects of the invention, as will become apparent from the description, are attained by the subject-matter of the independent claims. Some of the preferred embodiments of the present invention form the subject-matter of the dependent claims.

The present inventors have surprisingly found that after treatment of cells with a substance which is known to prime cells for a stress response the respiration activity of the cells increases, while it does not increase upon treatment of cells with substances which are known not to prime cells for a stress response. The increased respiration activity, in particular the oxygen transfer rate, can be detected by known methods and apparatuses such as the respiration activity monitoring system (RAMOS). Without wishing to be bound by a particular theory, it is hypothesized that by increasing the respiration activity the cells prepare for the synthesis of hydrogen peroxide which is a known mediator of plant defense mechanisms.

The above finding can be used to identify further substances which are able to prime cells for a stress response by determining the respiration activity, in particular the oxygen transfer rate, of the cells. The method of the present invention can be used with any interesting cell type and the substances identified can then be directly used in combination with this cell type. This distinguishes the present method from other methods in which a model cell type is used and the findings obtained with the model cell type then have to be transferred to the cell type of interest. Further, as the method of the present invention involves the continuous monitoring of the respiration activity, more information on the interaction of the candidate substance with the cell can be obtained.

Accordingly, the present invention provides a method for identifying substances which prime cells for a stress response comprising the steps of:

- inoculating cells in a suitable culture medium and culturing them in said medium;
- adding one or more candidate substances to the culture medium thereby treating the cells with said candidate substance; and
- measuring the respiration activity in the cells treated with the candidate substance and in control cells not treated with the candidate substance,
wherein an increase in the respiration activity in the cells treated with the candidate substance compared to the control cells indicates that the candidate substance primes the cells for a stress response.

Preferably, the respiration activity is determined by measuring the oxygen transfer rate.
Preferably, the cells are plant or mammalian cells.
The present invention also provides a method for identifying substances which prime plant cells for a stress response comprising the steps of:

- inoculating plant cells in a suitable culture medium and culturing them in said medium;
- adding one or more candidate substances to the culture medium thereby treating the plant cells with said candidate substance; and
- measuring the oxygen transfer rate in the cells treated with the candidate substance and in control cells not treated with the candidate substance,
wherein an increase in the oxygen transfer rate in the cells treated with the candidate substance compared to the control cells indicates that the candidate substance primes the plant cells for a stress response.

Also preferably, the candidate substance is added 24 to 120 hours after inoculation and/or when the oxygen transfer rate is about twice the oxygen transfer rate at the time of inoculation.
Also preferably, the cells are cultured in suspension.
In a further preferred embodiment the method further comprises, after the addition of the candidate substance, subjecting the cells to a compound eliciting a stress response.
In another embodiment the cells are subjected to the compound eliciting a stress response 6 to 48 hours after the addition of the candidate substance.
In a preferred embodiment a difference in respiration activity between cells treated with the candidate substance and untreated control cells is quantified using the formula I:

$$oxygen\ consumption\ (t_1 - t_4)\ [\%] = \left( \frac{\int_{t1}^{t4} OTR_{+priming\ compound}}{\int_{t1}^{t4} OTR_{no\ additives}} - 1 \right) \cdot 100$$

wherein $\int_{t1}^{t4} OTR_{+priming\ compound}$ is the area under the curve for cells treated with the candidate substance, $\int_{t1}^{t4} OTR_{no\ additives}$ is the area under the curve for the untreated control cells, t1 is the point in time at which the candidate substance is added and t4 is the point in time after addition of the candidate substance at which the oxygen transfer rate of the cells treated with the candidate substance is the same as the oxygen transfer rate of the untreated cells.

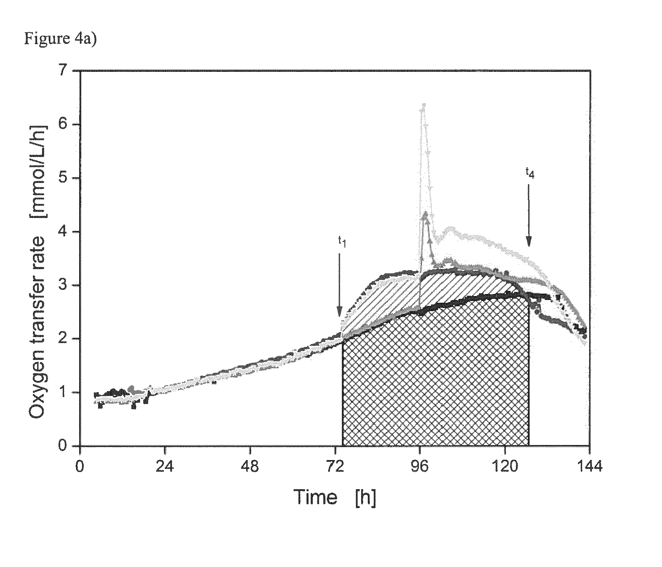

The areas under the curve which have to be determined for this analysis method are shown in Figure 4a.
Alternatively or additionally, a difference in respiration activity between cells treated with the candidate substance and untreated control cells is quantified using the formula II:

$$oxygen\ consumption\ (t_1 - t_2)\ [\%] = \left( \frac{\int_{t1}^{t2} OTR_{+priming\ compound}}{\int_{t1}^{t2} OTR_{no\ additives}} - 1 \right) \cdot 100$$

wherein $\int_{t1}^{t2} OTR_{+priming\ compound}$ is the area under the curve for cells treated with the candidate substance, $\int_{t1}^{t2} OTR_{no\ additives}$ is the area under the curve for the untreated control cells, t1 is the point in time at which the candidate substance is added and t2 is the point in time at which the cells are subjected to a compound eliciting a stress response.

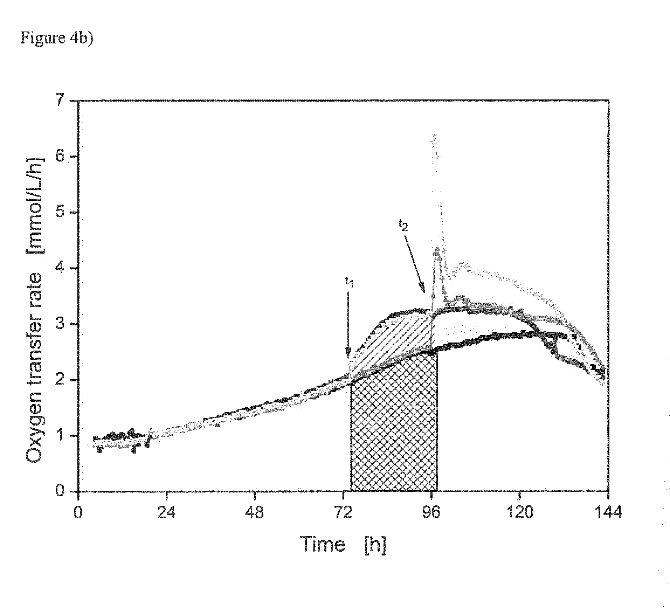

The areas under the curve which have to be determined for this analysis method are shown in Figure 4b.

Alternatively or additionally, a relative increase of the respiration activity upon subjecting the cells to the compound eliciting a stress response in cells treated with the candidate substance in comparison to the untreated control cells is quantified using the formula III:

$$Relative\ \Delta OTR_{max}[\%] = \left( \frac{\Delta OTR_{max}1}{\Delta OTR_{max}2} - 1 \right) \cdot 100$$

wherein $\Delta OTR_{max}1$ is the difference of the oxygen transfer rate between the peak oxygen transfer rate after adding the compound eliciting a stress response and the oxygen transfer rate at the time at which the compound eliciting a stress response is added in cells treated with the candidate substance and a compound eliciting a stress response and $\Delta OTR_{max}2$ is the difference of the oxygen transfer rate between the peak oxygen transfer rate after adding the compound eliciting a stress response and the oxygen transfer rate at the time at which the compound eliciting a stress response is added in the control cells only treated with the compound eliciting a stress response.

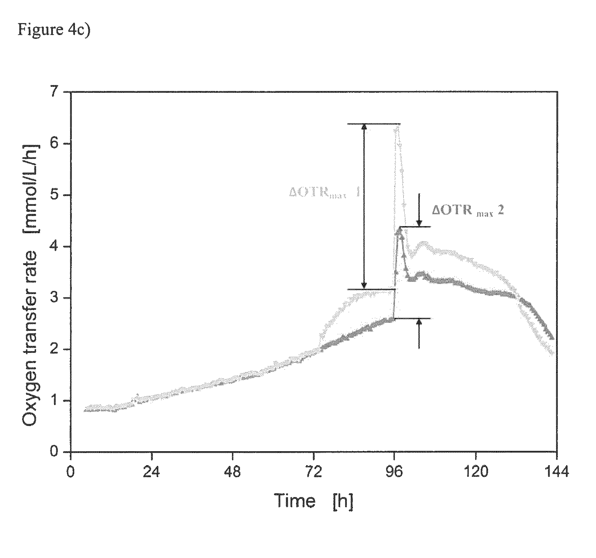

This quantification method is illustrated in Figure 4c.

Alternatively or additionally, a difference in respiration activity between cells treated with the candidate substance and control cells is quantified using the formula IV:

$$oxygen\ consumption\ (t_2 - t_3)\ [\%] = \left( \frac{\int_{t2}^{t3} OTR_{+priming\ compound/+elicitor}}{\int_{t2}^{t3} OTR_{+elicitor}} - 1 \right) \cdot 100$$

wherein $\int_{t2}^{t3} OTR_{+priming\ compound/+elicitor}$ is the area under the curve for cells treated with the candidate substance and subjected to the compound eliciting a stress response, $\int_{t2}^{t3} OTR_{+elicitor}$ is the area under the curve for control cells subjected to the compound eliciting a stress response, t2 is the point in time at which the cells are subjected to the compound eliciting a stress response and t3 is the point in time 12 to 48 hours after subjecting the cells to the compound eliciting a stress response.

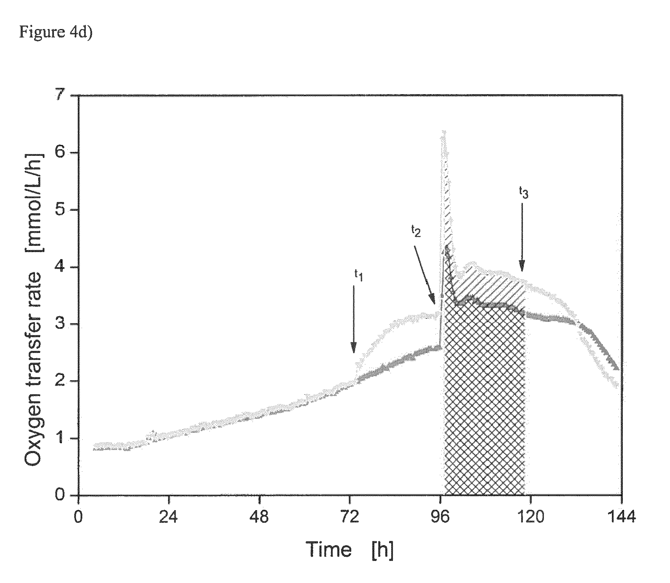

This quantification method is illustrated in Figure 4d.

Alternatively or additionally, a difference in respiration activity between cells treated with the candidate substance and control cells is quantified by calculating a linear regression of the oxygen transfer rate curve for the time before adding the candidate substance and an exponential regression of the oxygen transfer rate curve for the time after adding the candidate substance and determining the tangent slope of the exponential regression at the interception of the linear and exponential regression. Alternatively, the ratio of the slope of the tangent of the exponential regression to the slope of the linear regression is calculated according to the formula VI:

$$Ratio\ [-] = \frac{a \cdot b \cdot e^{a(c-t)}}{m}$$

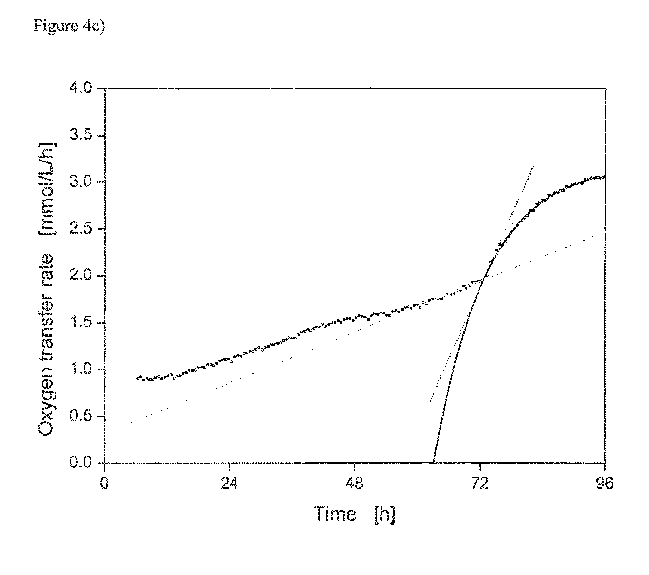

These quantification methods are illustrated in Figure 4e.

The eukaryotic cells may be plant or animal cells, preferably the plant cells are from *Petroselinum crispum, Triticum aestivum, Glycine max, Solanum tuberosum, Oryza sativa* or *Zea mays.*

Another aspect of the invention relates to the use of a respiration activity monitoring system for the identification of substances which prime eukaryotic cells for a stress response.

**BRIEF DESCRIPTION OF THE DRAWINGS**

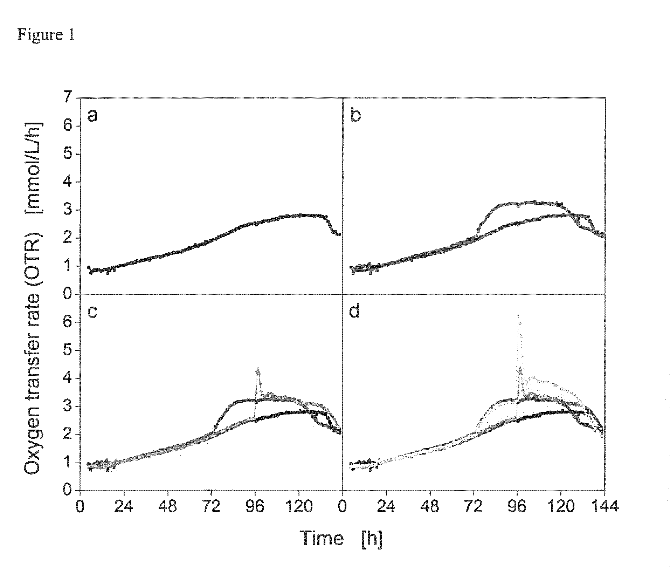

Figure 1: Oxygen transfer rate in *Petroselinum crispum* cells in different physiological states

a) Oxygen transfer rate in untreated control cells

b) Oxygen transfer rate in cells treated with 200 $\mu$M salicylic acid as defense priming compound compared to untreated cells (in addition to the oxygen transfer rate of a))

c) Oxygen transfer rate in cells treated with 200 $\mu$M salicylic acid as defense priming compound or 1 nM Pep 13 as a compound eliciting a stress response (in addition to the oxygen transfer rates of a) and b))

d) Oxygen transfer rate in cells treated with 200 $\mu$M salicylic acid as defense priming compound and 1 nM Pep13 as a compound eliciting a stress response (in addition to the oxygen transfer rates of a) to c))

The graph with the black rectangles depicts the untreated culture, the graph with the dark grey circles depicts the culture treated with the defense priming substance, the graph with the grey triangles showing up depicts the culture treated with the compound eliciting a stress response and the graph with the light grey triangles showing down depicts the culture treated with both the defense priming substance and the compound eliciting a stress response Pep-13.

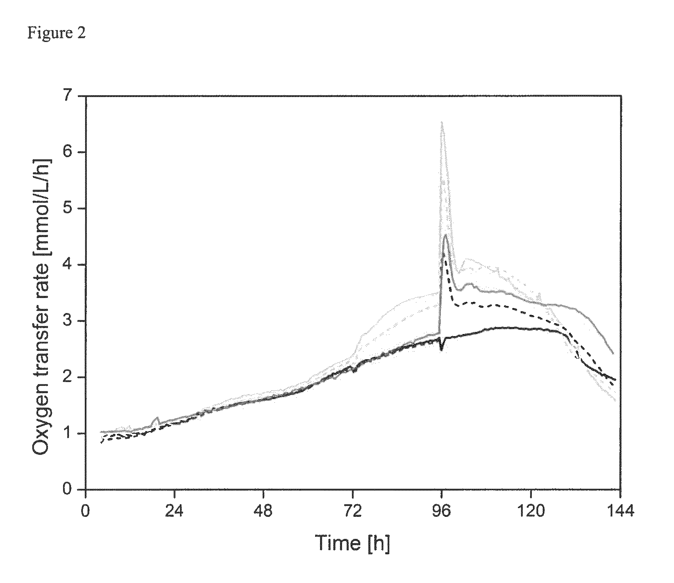

Figure 2: Oxygen transfer rate in cells treated with the known defense priming compounds salicylic acid and 4-chlor salicylic acid compared to the oxygen transfer rate in cells treated with the compound 3-hydroxybenzoic acid which does not have defense priming activity.

The graph with the black line depicts the untreated culture, the graph with the dark grey line depicts the culture treated with 1 nm Pep 13 as the compound eliciting a stress response, the graph with the light grey line depicts the culture treated with both salicylic acid and 1 nm Pep13, the dashed light grey line depicts the culture treated with both 4-chlor salicylic acid and the 1 nm Pep13, and the dashed dark grey line depicts the culture treated with both 3-hydroxybenzoic acid and the 1 nm Pep13.

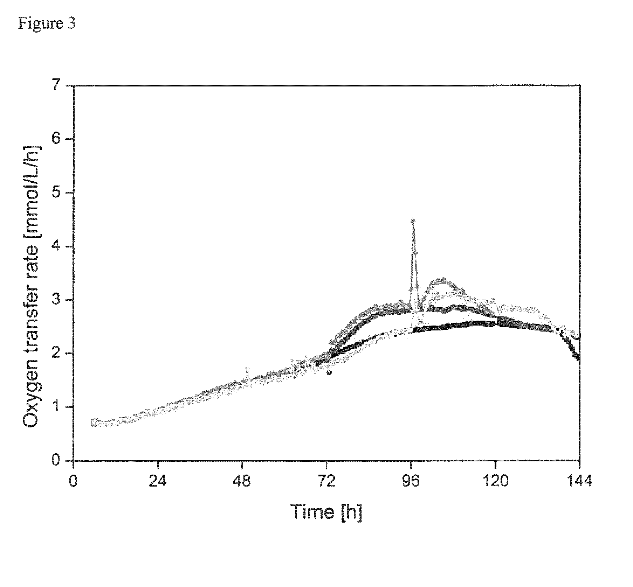

Figure 3: Oxygen transfer rate in cells treated with 200 $\mu$M salicylic acid as defense priming compound and 0.05 nM Pep13 as a compound eliciting a stress response

The graph with the black rectangles depicts the untreated culture, the graph with the dark grey circles depicts the culture treated with the defense priming substance, the graph with the light grey triangles showing down depicts the culture treated with the compound eliciting a stress response and the graph with the grey triangles showing up depicts the culture treated with both the defense priming substance and the compound eliciting a stress response Pep-13.

Figure 4: Different methods for calculating the increase in oxygen transfer rate after treatment with a defense priming compound

(a) Graph showing the area under the curve in *Petroselinum crispum* suspension cultures treated with the known defense priming substance salicylic acid (hatched area) 72 hours after inoculation and in control cells (sketched area) in the period after adding the defense priming substance ($t_1$) and the point in time at which the oxygen transfer rate of the treated cells is the same as the oxygen transfer rate of the untreated cells ($t_4$).

The graph with the black rectangles depicts the untreated culture, the graph with the dark grey circles depicts the culture treated with the defense priming substance, the graph with the grey triangles showing up depicts the culture treated with the compound eliciting a stress response and the graph with the light grey triangles showing down depicts the culture treated with both the defense priming substance and the compound eliciting a stress response Pep-13.

(b) Graph showing the area under the curve in *Petroselinum crispum* suspension cultures treated with the known defense priming substance salicylic acid (hatched area) and in untreated control cells (sketched area) in the period after adding the defense priming substance ($t_1$) and the point in time at which the cells are subjected to the compound eliciting a stress response Pep-13 ($t_2$).

The graph with the black rectangles depicts the untreated culture, the graph with the dark grey circles depicts the culture treated with the defense priming substance, the graph with the grey triangles showing up depicts the culture treated with the compound eliciting a stress response and the graph with the light grey triangles showing down depicts the culture treated with both the defense priming substance and the compound eliciting a stress response.

(c) Graph showing the increase of the oxygen transfer rate in *Petroselinum crispum* suspension cultures treated with the known defense priming substance salicylic acid and subjected to the compound eliciting a stress response Pep-13 ($\Delta OTR_{max}1$) and in cells only subjected to the compound eliciting a stress response Pep-13 ($\Delta OTR_{max}2$).

The graph with the grey triangles showing up depicts the culture treated with the compound eliciting a stress response and the graph with the light grey triangles showing down depicts the culture treated with both the defense priming substance and the compound eliciting a stress response.

(d) Graph showing the area under the curve in *Petroselinum crispum* suspension cultures treated with the known defense priming substance salicylic acid (hatched area) and in control cells (sketched area) in the period after subjecting the cells to the compound eliciting a stress response Pep-13 ($t_2$) and 24 hours later ($t_3$).

The graph with the grey triangles showing up depicts the culture treated with the compound eliciting a stress response and the graph with the light grey triangles showing down depicts the culture treated with both the defense priming substance and the compound eliciting a stress response.

(e) Graph showing the linear and exponential regression of the oxygen transfer rate curve over time in *Petroselinum crispum* suspension cultures.

The light grey line represents the linear regression of the oxygen transfer rate curve in a period of ten hours before adding the known defense priming substance salicylic acid. The dark grey line represents the exponential regression of the oxygen transfer rate curve in a period of 22 hours after adding the known defense priming substance salicylic acid. The dotted line shows the slope of the tangent of the exponential regression at the intersection of the linear and exponential regression.

## DETAILED DESCRIPTION OF THE INVENTION

The present invention as illustratively described in the following may suitably be practiced in the absence of any element or elements, limitation or limitations, not specifically disclosed herein.

The present invention will be described with respect to particular embodiments, but the invention is not limited thereto, but only by the claims.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated.

The term "priming for a stress response" refers to the induction of a physiological state that enables the primed cells to respond to very low levels of a stimulus, preferably an abiotic or biotic stress, in a faster and/or stronger manner than non-primed cells. Thus, primed cells show faster and/or stronger activation of stress responses than non-primed cells when challenged by biotic or abiotic stress after priming.

Stress responses are the reaction of a cell or organism to biotic or abiotic stress and include, but are not limited to, enhanced transcription of defense genes encoding enzymes such as PAL1, PAL2, 4CL, C4H and transcription factors such as WRKY29, WRKY6 and WRKY53 and enhanced production of antimicrobial substances such as phytoalexines. Biotic stress includes the infection of a cell with a pathogen such as fungi, bacteria, viruses, insects and nematodes. Abiotic stress includes drought, heat, cold and radiation stress.

Substances which prime eukaryotic cells, in particular plant cells, for a stress response prepare the immune system of the cells for future stress. In plants which have been primed for a stress response, the stress response occurs earlier and is stronger, leading to a more effective resistance than in plants which have not been primed for a stress response.

Substances which are known to prime plant cells for a stress response include benzo(1,2,3)thiadiazole-7-carbothioic acid S-methyl ester (BTH), salicylic acid (SA) or acetyl salicylic acid (aspirin).

Candidate substances are substances which should be tested for their ability to prime cells for a stress response and include natural and synthetic substances. The term "candidate substances" is also intended to include bacteria and other microorganisms which may secrete substances which prime cells for a stress response. It has been shown that the colonization of Arabidopsis roots with *Pseudomonas fluorescens* bacteria primes the plant for a response to pathogens (Hase et al. (2003) Physiological and Molecular Plant Pathology 62: 219-226). The candidate substances can be tested in different concentrations to determine the minimum concentration which is required for priming the cells for a stress response.

As a positive control, another set of cells may in parallel be treated with one or more substances, from which it is known that they prime cells for a stress response, such as benzo(1,2,3)thiadiazole-7-carbothioic acid S-methyl ester (BTH), salicylic acid (SA), 4-chloro salicylic acid or acetyl salicylic acid (aspirin), either alone or in combination with a compound eliciting a stress response.

As a negative control, another set of cells may in parallel be treated with one or more substances, from which it is known that they do not prime cells for a stress response, such as 3-hydroxy benzoic acid.

Those candidate substances which lead to a significant increase in the respiration activity in the treated cells in comparison to the untreated control cells are substances which prime the cells for a stress response.

After identifying substances which are capable of priming a stress response by the method of the present invention, it can be confirmed that these substances prime the cells for a stress response by treatment of plants with this substance and then contacting the plants with pathogen or abiotic stress and measuring the stress response. It is confirmed that a substance primes cells for a stress response, if after treatment with the substance and contacting the plants with pathogen or abiotic stress the stress response in the plant treated with the substance occurs earlier and/or is stronger than in a plant not treated with the substance.

If the cells used in the method of the present invention are parsley (*Petroselinum crispum*) cells, the priming effect of the candidate substance can also be confirmed by detection of fluorescent substances which are released from the parsley cells upon treatment with a compound eliciting a stress response as described in Siegrist et al. (1998) Physiol. Mol. Plant Pathol. 53(4): 223-238. The fluorescence is measured 6 to 48 hours, preferably 12 to 40 hours, more preferably 16 to 36 hours, even more preferably 20 to 32 hours and most preferably 24 hours after addition of the compound eliciting a stress response.

The cells which are used in the method of the present invention may be plant or animal cells, such as mammalian cells. Preferably, plant cells are used. The plant cells may be derived from a monocotyledonous or dicotyledonous plant.

Examples of monocotyledonous plants are plants belonging to the genera Avena (oat), Triticum (wheat), Secale (rye), Hordeum (barley), Oryza (rice), Panicum, Pennisetum, Setaria, Sorghum (millet), Zea (maize), and the like.

Dicotyledonous plants comprise, *inter alia,* Arabidopsis, cotton, legumes, like leguminous plants and in particular alfalfa, soybean, rape, canola, tomato, sugar beet, potato, ornamental plants, and trees. Further dicotyledonous plants can comprise fruit (in particular apples, pears, cherries, grapes, citrus, pineapple, and bananas), pumpkin, cucumber, wine, oil palms, tea shrubs, cacao trees, and coffee shrubs, tobacco, sisal, as well as, with medicinal plants, rauwolfia and digitalis.

Particularly preferred for use in the method of the present invention are cells from agronomically important plant species, since the substances identified by the method of the present invention can then directly be used for the treatment of plants of this species. Also particularly preferred are cells from plant species which show a uniform growth profile such as *Petroselinum crispum.*

Most preferably, the cells are from *Petroselinum crispum, Triticum aestivum, Solanum tuberosum, Oryza sativa, Glycine max* or *Zea mays.*

Suitable mammalian cells include, but are not limited to, human, mouse, rat, hamster, bovine and porcine cells. Preferably the cells are rodent or human cells. Examples of suitable mammalian cells include HeLa, NIH3T3, CHO and 293 cells.

The term "inoculating" means that the cells which are used in the method of the present invention are brought into contact with the culture medium.

The term "culturing" is intended to mean that the cells are held under conditions such as medium, temperature and pH which allow the maintenance and proliferation of the cells. For plant cells such as *Petroselinum crispum* cells, the medium may be modified Gamborg B5 medium and the temperature may be 25°C. In the method of the present invention the cells are preferably grown in suspension culture, i.e. without being attached to a substrate. More preferably, the cells are grown in shaking flasks, such as a shake flask with a nominal volume of 100 to 500 mL with a filling volume of 1/20 to 1/4 of the nominal volume, a shaking diameter of 12.5 to 100 mm and a shaking frequency of 150 to 400 rpm. Most preferably, the cells are grown in a shake flask with a nominal volume of 250 mL, a filling volume of 50 mL, a shaking diameter of 5 cm and a shaking frequency of 180 rpm.

"Culture medium" is used for the maintenance of cells in culture *in vitro.* For some cell types, the medium may also be sufficient to support the proliferation of the cells in culture. A culture medium typically provides nutrients such as energy sources, amino acids and inorganic ions. Additionally, it may contain a dye like phenol red, sodium pyruvate, several vitamins, free fatty acids, antibiotics, anti-oxidants, minerals and trace elements.

For culturing plant cells a number of media is available, including CHU ($N_6$) medium, Gamborg B5 medium, Murashige & Skoog Medium (MS), Murashige & Skoog Modified Medium and Schenk & Hildebrandt Medium (SH). These media are well-known to a person skilled in the art and may be purchased from companies such as Sigma-Aldrich (Deisenhofen, Germany) and PhytoTechnology Laboratories (Shawnee Mission, USA).

For culturing mammalian cells any standard medium such as Iscove's Modified Dulbecco's Media (IMDM), alpha-MEM, Dulbecco's Modified Eagle Media (DMEM), RPMI Media and McCoy's Medium may be used. These media are well-known to a person skilled in the art and may be purchased from companies such as Cambrex (East Rutherford, USA), Invitrogen (San Diego, USA) and Sigma-Aldrich (Deisenhofen,Germany).

The term "respiration activity" refers to the activity of the cells in culture which consumes or produces respiratory gases such as oxygen and carbon dioxide, respectively. The respiration activity can be described by different parameters such as the oxygen transfer rate (OTR), the carbon dioxide transfer rate (CTR), dissolved oxygen partial tension (DOT) and the respiration quotient (RQ). In particular, within the method of the present invention the determination of the respiration activity includes the determination of the oxygen transfer rate.

The oxygen transfer rate is a parameter for describing the oxygen consumption of a cell culture. If the oxygen transfer rate is determined with the RAMOS technology, it can be calculated according to the simplified formula:

$$\text{OTR [mol/L/h]} = \Delta pO_2/\Delta t * V_G/R/T/V_L,$$

wherein :

$\Delta pO_2$ is the difference of gas phase oxygen partial pressure (bar)

$\Delta t$ is the time of the measuring phase (h)

$V_G$ is the head space gas volume (L)

R is the gas constant (bar l/mol/K)

T is the temperature (K)

$V_L$ is the liquid filling volume of the shake flask (L)

The carbon dioxide transfer rate describes the molar production of carbon dioxide during the cultivation. If the carbon dioxide transfer rate is determined with the RAMOS technology, it can be calculated according to the simplified formula:

$$\text{CTR [mol/L/h]} = \Delta pCO_2/\Delta t * V_G/RTV_L,$$

wherein

$\Delta pCO_2$ is the difference of gas phase carbon dioxide partial pressure (bar)

$\Delta t$ is the time of the measuring phase (h)

$V_G$ is the head space gas volume (L)

R is the gas constant (bar l/mol/K)

T is the temperature (K)

$V_L$ is the liquid filling volume of the shake flask (L)

The respiratory quotient describes the ratio between CTR and OTR and is, therefore, calculated according to the formula:

$$RQ = CTR/OTR$$

The respiration activity can be determined by any method known in the art. Preferably, the respiration activity is determined by a respiration activity monitoring system (RAMOS). This system is described in German patent application DE 44 15 444 and European patent application EP 0 905 229 A2 as well as in Anderlei and Buechs (2001) Biochemical Engineering Journal 7: 157-162 and Anderlei et al. (2004) Biochemical Engineering Journal 17: 187-194. A suitable RAMOS device is commercially available from HiTec Zang GmbH (Herzogenrath, Germany) and from Kuhner AG (Birsfelden, Switzerland).

In the determination of the respiration activity with RAMOS, a measuring cycle is continuously repeated during fermentation. This measuring cycle comprises a measuring phase and a rinsing phase. During the rinsing phase, air is flushed through the measuring flask at a specific flow rate. At the beginning of the measuring phase, inlet and outlet valves of the measuring flask are closed. Respiration of the cells leads to a decrease in the oxygen partial pressure and to an increase in the carbon dioxide partial pressure in the gas headspace of the measuring flask. These partial pressures are monitored by an oxygen sensor and a differential pressure sensor. In addition, a carbon dioxide sensor may be used. Assuming linear changes in the measuring phase, a computer calculates the oxygen transfer rate and the carbon dioxide transfer rate according to the formulas provided above. After the measuring phase, the valves are opened again, and the next measuring cycle starts. Before each measuring phase, the sensors are calibrated using the known steady state gas composition to compensate for signal drift. This calibration is described in Anderlei and Büchs (2001) Biochemical Engineering Journal 7: 157-162, German patent application DE 44 15 444 and Anderlei et al. (2004) Biochemical Engineering Journal 17: 187-194.

In the RAMOS method, the measuring cycle can be adjusted to the respiration behavior of the cells which are investigated. If these cells have a low overall respiration activity the measuring phase has to be longer than for cells with a higher respiration activity. For example, for rapidly growing bacterial and yeast cells the measuring cycle is typically five to ten minutes and for slower growing plant or animal cells the measuring cycle is typically 10 to 40 minutes.

Alternatively, the oxygen transfer rate may be determined by measuring the oxygen mole fraction of exhaust oxygen gas and the carbon dioxide mole fraction of exhaust carbon dioxide gas (see Biochemical Engineering Fundamentals by James E. Bailey and David F. Ollis, Second Edition, ISBN 0-07-003212-2, Page 472, equation 8.25 and Knoll et al. (2005) Adv. Biochem. Engin/Biotechnol 92: 77-99, equation 21). For oxygen measurement e.g. paramagnetic oxygen analyzers and for carbon dioxide infrared analyzers are applied. In this case, the oxygen transfer rate is calculated using the formula:

$$OTR = \frac{q_{in}}{V_{mo}} \cdot \left( y_{O2,in} - \frac{1 - y_{O2,in} - y_{CO2,in}}{1 - y_{O2,out} - y_{CO2,out}} \cdot y_{O2,out} \right)$$

wherein

OTR is the oxygen mass transfer rate [mol/L/h]

$q_{in}$ is the volume specific aeration rate at standard conditions (0°C, 1.01325 bar) [vvm]

$V_{mo}$ is the molar gas volume (22.414 L/mol) at 0°C, 1.01325 bar [L/mol]

$y_{O2}$, in is the oxygen mole fraction of inlet gas (with air: 0.2095) [mol/mol]

$y_{O2,out}$ is the oxygen mole fraction of exhaust gas [mol/mol]

$y_{CO2,in}$ is the carbon dioxide mole fraction of inlet gas (with air: 0.00035) [mol/mol

$y_{CO2,out}$ is the carbon dioxide mole fraction of exhaust gas [mol/mol]

The volume specific aeration rate may be determined with conventional methods.

In a third alternative the oxygen transfer rate is calculated from the mole fraction of dissolved oxygen according to the following formula:

$$OTR = \frac{k_L a \cdot L_{O_2} \cdot p_{abs} \cdot (y_{O_2,in} - y_L)}{1 + \frac{k_L a \cdot L_{O_2} \cdot p_{abs} \cdot V_{mo}}{q_{in}}}$$

wherein

OTR is the oxygen mass transfer rate [mol/L/h]

$k_L a$ is the volumetric mass transfer coefficient [1/h]

$L_{O2}$ is the $O_2$-solubility [mol/L/bar]

$p_{abs}$ is the absolute pressure in the system (at half of the liquid height) [bar]

$y_{O2,in}$ is the $O_2$-mole fraction in the liquid at the gas/liquid phase boundary [mol/mol]

$y_L$ is the $O_2$-mole fraction that is equivalent to the dissolved oxygen concentration in the liquid ($SO_2/100 \cdot y_{cal}$) [mol/mol]

$SO_2$ is the signal of the DOT electrode [%]

$y_{cal}$ is the $O_2$-mole-fraction at which the DOT-probe was calibrated to 100 % [mol/mol]

$q_{in}$ is the volume specific aeration rate [1/h]

The oxygen solubility in the culture medium ($L_{O2}$) and the volume specific aeration rate ($q_{in}$) can be determined by conventional methods. The volumetric mass transfer coefficient ($k_L a$) depends on the operating conditions of the bioreactor used and the composition of the culture medium and can be determined by methods known to the skilled person.

In the method of the present invention the respiration activity of the cells treated with the candidate substance is compared with the respiration activity of control cells not treated with the candidate substance. These control cells can be cells in the culture medium which are not treated with any substance, if the treated cells are only treated with the candidate substance, or they can be cells subjected to a compound eliciting a stress response, if the cells treated with the candidate substance are also subjected to said compound eliciting a stress response.

A candidate substance primes cells for a stress response, if the cells treated with the candidate substance have a higher respiration activity, in particular a higher oxygen transfer rate, than the cells not treated with the candidate substance.

The candidate substance is added to the culture medium 24 to 120 hours after inoculation, preferably 36 to 100 hours, more preferably 48 to 90 hours, even more preferably 60 to 80 hours and most preferably 72 hours after inoculation and/or when the oxygen transfer rate is about twice the oxygen transfer rate at the time of inoculation. At this point in time, the oxygen transfer rate in plant cells is preferably 0.5 to 4 mmol/L/h, more preferably 0.8 to 3.5 mmol/L/h and most preferably 1 to 3 mmol/L/h and in animal cells the oxygen transfer rate is preferably 0.1 to 0.8 mmol/L/h, more preferably 0,16 to 0.7 mmol/L/h and most preferably 0.2 to 0.6 mmol/L/h.

In one embodiment of the present invention the cells may be subjected to a compound eliciting a stress response. The elicitor of a stress response is any substance, organism or environmental condition which elicits a stress response within a cell, in particular a plant cell. Abiotic elicitors for plant cells include drought, heat, cold and radiation stress. Biotic elicitors for plant cells include pathogens such as fungi, bacteria, nematodes, insects and viruses.

The compound eliciting a stress response may also be a pathogen-associated molecular pattern (PAMP) which is a small molecular motif present within pathogens, but not in the recipient organism. It is recognized by toll-like receptors and other pattern recognition receptors in plant and animal cells and then induces a defense response within the cell. Suitable PAMPs are known to the expert and include Pep-13 (Nürnberger et al. (1994) Cell 78: 449-460), NPP1 (Fellbrich et al. (2002) Plant J. 32(3): 375-390), flg22 (Felix et al. (1999) Plant J. 18(3): 265-276), CBEL (Villalba-Mateos et al. (1997) Mol. Plant Microbe Interact. 10: 1045-1053), AsES (Chalfoun et al. (2013) J. Biol. Chem. 288(20): 14098-14113) and TLKGE (Rotblat et al. (2002) Plant J. 32(6): 1049-1055).

The cells are subjected to the compound eliciting a stress response 6 to 48 hours, preferably 12 to 40 hours, more preferably 16 to 36 hours, even more preferably 20 to 32 hours and most preferably 24 hours after addition of the candidate substance.

Any difference in the respiration activity, preferably the oxygen transfer rate, between the cells treated with the candidate substance and the untreated control cells can be visualized by plotting preferably the oxygen transfer rate measured in mmol/L/h (on the y axis) against the culture time (on the x axis) for the cells treated with the candidate substance and the untreated control cells. Figures 1 to 4 show that the treatment of cells with substances from which it is known that they prime cells for a stress response increases the oxygen transfer rate in a characteristic manner, whereas it is not increased with substances from which it is known that they do not prime cells for a stress response.

The difference in respiration activity, preferably the oxygen transfer rate, between the cells treated with the candidate substance and the untreated control cells can be quantified by several calculations, used either alone or in combination. Generally, it is sufficient, if a candidate substance leads to a significant increase of the oxygen transfer rate in any of these calculations, but a combination of two or more calculation methods may provide more detailed information.

A first possibility to quantify any difference in respiration activity is to determine the relative oxygen consumption as the relative area between the difference of the oxygen transfer rate in the cells treated with the candidate substance and the oxygen transfer rate in the untreated control cells in relation to the area under the curve for the untreated control cells in the period after adding the candidate substance and the point in time at which the oxygen transfer rate of the treated cells is the same as the oxygen transfer rate of the untreated cells. This calculation is illustrated in Figure 4a, wherein the relative area between the hatched area and the checked area is determined.

The relative oxygen consumption is determined using the following formula I:

$$oxygen\ consumption\ (t_1 - t_4)\ [\%] = \left( \frac{\int_{t1}^{t4} OTR_{+priming\ compound}}{\int_{t1}^{t4} OTR_{no\ additives}} - 1 \right) \cdot 100$$

wherein $\int_{t1}^{t4} OTR_{+priming\ compound}$ is the area under the curve for cells treated with the candidate substance, $\int_{t1}^{t4} OTR_{no\ additives}$ is the area under the curve for the untreated control cells, t1 is the point in time at which the candidate substance is added and t4 is the point in time at which the oxygen transfer rate of the treated cells is the same as the oxygen transfer rate of the untreated cells.

A candidate substance is considered to prime cells for a stress response, if the relative oxygen consumption calculated using the above formula I is at least 5%, preferably at least 8%, more preferably at least 10 %, even more preferably at least 12% and most preferably at least 15%.

A second possibility to quantify any difference in respiration activity is to determine the relative oxygen consumption as the relative area between the difference of the oxygen transfer rate in the cells treated with the candidate substance and the oxygen transfer rate in the untreated control cells in relation to the area under the curve for the untreated control cells in the period after adding the candidate substance until the cells are subjected to a compound eliciting a stress response. This calculation is illustrated in Figure 4b, wherein the relative area between the hatched area and the checked area is determined.

The relative oxygen consumption is determined using the following formula II:

$$oxygen\ consumption\ (t_1 - t_2)\ [\%] = \left( \frac{\int_{t1}^{t2} OTR_{+priming\ compound}}{\int_{t1}^{t2} OTR_{no\ additives}} - 1 \right) \cdot 100$$

wherein $\int_{t1}^{t2} OTR_{+priming\ compound}$ is the area under the curve for cells treated with the candidate substance, $\int_{t1}^{t2} OTR_{no\ additives}$ is the area under the curve for the untreated control cells, t1 is the point in time at which the candidate substance is added and t2 is the point in time at which the cells are subjected to a compound eliciting a stress response.

A candidate substance is considered to prime cells for a stress response, if the relative oxygen consumption calculated using the above formula II is at least 5%, preferably at least 10%, more preferably at least 13 %, even more preferably at least 15% and most preferably at least 20%.

A third possibility to quantify any difference in respiration activity is to determine a relative increase of the respiration activity, preferably the oxygen consumption, upon subjecting the cells to a compound eliciting a stress response in cells treated with the candidate substance and control cells. This calculation is illustrated in Figure 4c, wherein $\Delta OTR_{max}1$ is the increase of the oxygen transfer rate in cells treated with both the candidate substance and the compound eliciting a stress response and $\Delta OTR_{max}2$ is the increase of the oxygen transfer rate in the control cells treated with the compound eliciting a stress response.

The relative increase in oxygen consumption can be calculated using the following formula III:

$$Relative\ \Delta OTR_{max} = \left( \frac{\Delta OTR_{max}1}{\Delta OTR_{max}2} - 1 \right) \cdot 100$$

wherein $\Delta OTR_{max}1$ is the difference of the oxygen transfer rate between the peak oxygen transfer rate after adding the compound eliciting a stress response and the oxygen transfer rate at the time at which the compound eliciting a stress response is added in cells treated with both the candidate substance and a compound eliciting a stress response and $\Delta OTR_{max}2$ is the difference of the oxygen transfer rate between the peak oxygen transfer rate after adding the compound eliciting a stress response and the oxygen transfer rate at the time at which the compound eliciting a stress response is added in the control cells only treated with the compound eliciting a stress response.

A candidate substance is considered to prime cells for a stress response, if the relative increase in oxygen consumption calculated using the above formula III is at least 25%, preferably at least 35%, more preferably at least 45%, even more preferably at least 50% and most preferably 60%.

A fourth possibility to quantify any difference in respiration activity is to determine the relative oxygen consumption as the relative area between the difference of the oxygen transfer rate in the cells treated with both the candidate substance and a compound eliciting a stress response and the oxygen transfer rate in the cells treated only with the compound eliciting a stress response in relation to the area under the curve for the cells treated only with the compound eliciting a stress response in a period of 12 to 48 hours, preferably 15 to 42 hours, more preferably 18 to 36 hours, even more preferably 20 to 30 hours and most preferably 24 hours after adding the compound eliciting a stress response. This calculation is illustrated in Figure 4d, wherein the relative oxygen consumption between the hatched area and the checked area is determined.

The relative oxygen consumption is determined using the following formula IV:

$$oxygen\ consumption\ (t_2 - t_3)\ [\%] = \left( \frac{\int_{t2}^{t3} OTR_{+priming\ compound/+elicitor}}{\int_{t2}^{t3} OTR_{+elicitor}} - 1 \right) \cdot 100$$

wherein $\int_{t2}^{t3} OTR_{+priming\,compound/+elicitor}$ is the area under the curve for cells treated with the candidate substance and subjected to the compound eliciting a stress response, $\int_{t2}^{t3} OTR_{+elicitor}$ is the area under the curve for cells subjected to the compound eliciting a stress response, t2 is the point in time at which the compound eliciting a stress response is added and t3 is the point in time 12 to 48 hours, preferably 15 to 42 hours, more preferably 18 to 36 hours, even more preferably 20 to 30 hours and most preferably 24 hours after adding the compound eliciting a stress response.

A candidate substance is considered to prime cells for a stress response, if the relative oxygen consumption calculated using the above formula IV is at least 10%, preferably at least 12%, more preferably at least 14 %, even more preferably at least 16% and most preferably at least 20%.

A fifth possibility to quantify any difference in respiration activity is to determine the slope of the oxygen transfer rate curve over time after adding the candidate substance or both before and after adding the candidate substance. This calculation is illustrated in Figure 4e and comprises two parts: the first part is a linear regression for a period before adding the candidate substance and the second part is an exponential regression for a period after adding the candidate substance. The period before adding the candidate substance for which the linear regression is calculated is 5 to 20 hours, preferably 6 to 17 hours, more preferably 7 to 15 hours, even more preferably 8 to 12 hours and most preferably 10 hours. The period after adding the candidate substance for which the exponential regression is calculated is 8 to 30 hours, preferably 12 to 28 hours, more preferably 15 to 26 hours, even more preferably 18 to 24 hours and most preferably 22 hours.

In this method, the data can be evaluated using the software Microsoft Excel and the constants d and m for the linear and a, b and c for the exponential regression, respectively, are determined according to the following formulas:

linear regression: $f(t) = m{\cdot}t+d$

exponential regression: $f(t) = b{\cdot}(1-e^{-\alpha(t-c)})$

Then, the slope of the tangent of the exponential regression at the interception of the linear and exponential regression is determined by the formula V:

$$\frac{\partial}{\partial x}\left(b \cdot \left(1 - e^{-a(t-c)}\right)\right) = a \cdot b \cdot e^{a(c-t)}$$

A candidate substance is considered to prime cells for a stress response, if the tangent has an absolute slope of at least 0.075 mmol/L/h$^2$, preferably of at least 0.09 mmol/L/h$^2$, more preferably of at least 0.11 mmol/L/h$^2$, even more preferably of at least 0.14 mmol/L/h$^2$ and most preferably of at least 0.17 mmol/L/h$^2$

Alternatively, the ratio of the slope of the tangent of the exponential regression to the slope of the linear regression is calculated according to the formula VI:

$$Ratio\ [-] = \frac{a \cdot b \cdot e^{a(c-t)}}{m}$$

A candidate substance is considered to prime cells for a stress response, if the ratio is at least three, preferably at least four, more preferably at least five and most preferably at least six.

In the plotting of the oxygen transfer rate versus time of cells cultured under different conditions in one graph it may happen that due to practical reasons the curves from inoculating the cell culture until the addition of the candidate substance do not have the same values. In this case, it may be necessary to align the curves to have the same starting conditions for all conditions investigated. This can be done by adjusting the position of one or more curves on the y axis so that ultimately the curves are superimposed. To this end, the sum of all differences between all reading points in the period from inoculating the culture until the addition of the candidate substance is calculated. Then different values are added to the absolute y values of the curve to be aligned using a known iterative method such as Excel Solver, until the sum of the differences between all reading points is minimal.

The identification of substances which prime for a stress response by measuring respiratory activity is described in the following examples:

**EXAMPLES**

1. Increase of the oxygen transfer rate upon treatment with a substance which primes the cells for a stress response

12.5 ml of a suspension of *Petroselinum crispum* cells were inoculated in 250 ml shaking flasks containing 37.5 ml of modified Gamborg B5 medium and cultured at a temperature of 25°C at a shaking frequency of 180U/min and a shaking diameter of 5cm in a RAMOS device (HiTec Zang, Herzogenrath). During the culture the oxygen partial pressure of the gas phase in the head space of the flask was measured and the oxygen transfer rate was calculated therefrom.

The oxygen transfer rate of the untreated cultures is shown in Figure 1a.

72 hours after inoculation of the cells the known defense priming substance salicylic acid was added at a concentration of 200 $\mu$M. The treatment with salicylic acid led to an increase in the oxygen transfer rate in comparison to the untreated cells which increase lasted about 48 hours (see Figure 1b).

In another experiment, 1nM of Pep13 which elicits a stress response of *Petroselinum crispum* cells was added to the culture 96 hours after inoculation. The treatment with Pep 13 led to a fast increase in the oxygen transfer rate (see Figure 1c).

In yet another experiment, the cells were treated with 200 $\mu$M salicylic acid 72 hours after inoculation and with Pep13 96 hours after inoculation. The treatment with salicylic acid and Pep13 led to a stronger increase in the oxygen transfer rate than the treatment with Pep13 alone (see Figure 1d).

These data show that the treatment of cells with a substance which primes the cells for a stress response leads to an increase in the oxygen transfer rate in comparison to untreated cells and to cells only treated with a compound eliciting a stress response.

2. Specificity of the increase in the oxygen transfer rate

To determine whether the observed increase of the oxygen transfer rate is specific for substances which prime cells for a stress response, the cells were treated with another substance (4-chlorsalicylic acid) which primes the cells for a stress response and a substance (3-hydroxy benzoic acid) which does not prime cells for a stress response.

In parallel experiments, the cells were either treated with 200 $\mu$M salicylic acid, 200 $\mu$M 4-chlorsalicylic acid or with 200 $\mu$M 3-hydroxy benzoic acid 72 hours after inoculation and then in all experiments 1 nM Pep13 was added 96 hours after inoculation.

The results in Figure 2 show that only those substances which prime cells for a stress response, i.e. salicylic acid and 4-chlorsalicylic acid, lead to an increase in the oxygen transfer rate in comparison to untreated cells and to cells only treated with a compound eliciting a stress response. The substance 3-hydroxy benzoic acid which does not prime cells for a stress response led to the same increase in the oxygen transfer rate as the addition of Pep13 alone.

**Claims**

1. Method for identifying substances which prime cells for a stress response comprising the steps of:

   - inoculating cells in a suitable culture medium and culturing them in said medium;
   - adding one or more candidate substances to the culture medium thereby treating the cells with said candidate substance; and
   - measuring the respiration activity in the cells treated with the candidate substance and in control cells not treated with the candidate substance,
   wherein an increase in the respiration activity in the cells treated with the candidate substance compared to the control cells indicates that the candidate substance primes the cells for a stress response.

2. Method according to claim 1, wherein measuring the respiration activity comprises measuring the oxygen transfer rate.

3. Method according to any of the preceding claims, wherein the cells are plant or mammalian cells.

4. Method for identifying substances which prime plant cells for a stress response comprising the steps of:

   - inoculating plant cells in a suitable culture medium and culturing them in said medium;
   - adding one or more candidate substances to the culture medium thereby treating the plant cells with said candidate substance; and

- measuring the oxygen transfer rate in the cells treated with the candidate substance and in control cells not treated with the candidate substance,

wherein an increase in the oxygen transfer rate in the cells treated with the candidate substance compared to the control cells indicates that the candidate substance primes the plant cells for a stress response.

5. Method according to any of the preceding claims, wherein the candidate substance is added 24 to 120 hours after inoculation.

6. Method according to any of the preceding claims, wherein the cells are cultured in suspension.

7. Method according to any of claims 2 to 6, wherein the candidate substance is added when the oxygen transfer rate is about twice the oxygen transfer rate at the time of inoculation.

8. Method according to any of the preceding claims, further comprising, after the addition of the candidate substance, subjecting the cells to a compound eliciting a stress response.

9. Method according to claim 8, wherein the cells are subjected to the compound eliciting a stress response 6 to 48 hours after the addition of the candidate substance.

10. Method according to any of claims 2 to 9, wherein a difference in respiration activity between cells treated with the candidate substance and control cells is quantified using the formula I:

$$oxygen\ consumption\ (t_1 - t_4)\ [\%] = \left( \frac{\int_{t1}^{t4} OTR_{+priming\ compound}}{\int_{t1}^{t4} OTR_{no\ additives}} - 1 \right) \cdot 100$$

wherein $\int_{t1}^{t4} OTR_{+priming\ compound}$ is the area under the curve for cells treated with the candidate substance,

$\int_{t1}^{t4} OTR_{no\ additives}$ is the area under the curve for the untreated control cells, t1 is the point in time at which the candidate substance is added and t4 is the point in time after addition of the candidate substance at which the oxygen transfer rate of the cells treated with the candidate substance is the same as the oxygen transfer rate of the untreated cells.

11. Method according to any of claims 8 to 10, wherein a difference in respiration activity between cells treated with the candidate substance and control cells is quantified using the formula II:

$$oxygen\ consumption\ (t_1 - t_2)\ [\%] = \left( \frac{\int_{t1}^{t2} OTR_{+priming\ compound}}{\int_{t1}^{t2} OTR_{no\ additives}} - 1 \right) \cdot 100$$

wherein $\int_{t1}^{t2} OTR_{+priming\ compound}$ is the area under the curve for cells treated with the candidate substance,

$\int_{t1}^{t2} OTR_{no\ additives}$ is the area under the curve for the untreated control cells, t1 is the point in time at which the candidate substance is added and t2 is the point in time at which the cells are subjected to a compound eliciting a stress response.

12. Method according to any of claims 8 to 11, wherein a relative increase of the respiration activity upon subjecting the cells to the compound eliciting a stress response in cells treated with the candidate substance in comparison to the control cells is quantified using the formula III:

$$Relative\ \Delta OTR_{max}[\%] = \left(\frac{\Delta OTR_{max}1}{\Delta OTR_{max}2} - 1\right) \cdot 100$$

wherein $\Delta OTR_{max}1$ is the difference of the oxygen transfer rate between the peak oxygen transfer rate after adding the compound eliciting a stress response and the oxygen transfer rate at the time at which the compound eliciting a stress response is added in cells treated with the candidate substance and a compound eliciting a stress response and $\Delta OTR_{max}2$ is the difference of the oxygen transfer rate between the peak oxygen transfer rate after adding the compound eliciting a stress response and the oxygen transfer rate at the time at which the compound eliciting a stress response is added in the control cells only treated with the compound eliciting a stress response.

13. Method according to any of claims 8 to 12, wherein a difference in respiration activity between cells treated with the candidate substance and control cells is quantified using the formula IV:

$$oxygen\ consumption\ (t_2 - t_3)\ [\%] = \left(\frac{\int_{t2}^{t3} OTR_{+priming\ compound/+elicitor}}{\int_{t2}^{t3} OTR_{+elicitor}} - 1\right) \cdot 100$$

wherein $\int_{t2}^{t3} OTR_{+priming\ compound/+elicitor}$ is the area under the curve for cells treated with the candidate substance and subjected to the compound eliciting a stress response, $\int_{t2}^{t3} OTR_{+elicitor}$ is the area under the curve for control cells only subjected to the compound eliciting a stress response, t2 is the point in time at which the cells are subjected to the compound eliciting a stress response and t3 is the point in time 12 to 48 hours after subjecting the cells to the compound eliciting a stress response.

14. Method according to any of claims 8 to 13, wherein a difference in respiration activity between cells treated with the candidate substance and control cells is quantified by calculating a linear regression of the oxygen transfer rate curve for the time before adding the candidate substance and an exponential regression of the oxygen transfer rate curve for the time after adding the candidate substance and determining the tangent slope of the exponential regression at the interception of the linear and exponential regression and/or by calculating the ratio of the slope of the tangent of the exponential regression to the slope of the linear regression according to the formula VI:

$$Ratio\ [-] = \frac{a \cdot b \cdot e^{a(c-t)}}{m}$$

15. Method according to any of the preceding claims, wherein the cells are from *Petroselinum crispum, Triticum aestivum, Glycine max, Solanum tuberosum, Oryza sativa* or *Zea mays.*

16. Use of a respiratory activity monitoring system for the identification of substances which prime eukaryotic cells for a stress response.

Figure 1

Figure 2

Figure 3

Figure 4a)

Figure 4b)

Figure 4c)

Figure 4d)

Figure 4e)

Europäisches
Patentamt

European
Patent Office

Office européen
des brevets

# EUROPEAN SEARCH REPORT

Application Number

EP 14 18 2508

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BEESON C C ET AL: "A high-throughput respirometric assay for mitochondrial biogenesis and toxicity", ANALYTICAL BIOCHEMISTRY, ACADEMIC PRESS INC, NEW YORK, vol. 404, no. 1, 1 September 2010 (2010-09-01), pages 75-81, XP027104737, ISSN: 0003-2697 [retrieved on 2010-05-11] * figure 4 * | 1-3,5, 7-9,16 | INV. G01N33/50 |
| A | S SURESH ET AL: "Techniques for oxygen transfer measurement in bioreactors: a review", JOURNAL OF CHEMICAL TECHNOLOGY & BIOTECHNOLOGY, vol. 84, no. 8, 1 August 2009 (2009-08-01) , pages 1091-1103, XP055162225, ISSN: 0268-2575, DOI: 10.1002/jctb.2154 * page 1098, column 1, paragraph 2 - column 2, paragraph 2 * | 10-14 | |
| A | YOSHITERU NOUTOSHI ET AL: "Novel Plant Immune-Priming Compounds Identified via High-Throughput Chemical Screening Target Salicylic Acid Glucosyltransferases in Arabidopsis", THE PLANT CELL, AMERICAN SOCIETY OF PLANT BIOLOGISTS, US, [Online] vol. 24, no. 9, 1 September 2012 (2012-09-01), pages 3795-3804, XP002715479, ISSN: 1040-4651 Retrieved from the Internet: URL:http://dx.doi.org/10.1105/tpc.112> [retrieved on 2012-09-01] * figures 1,3 * | 1-16 | TECHNICAL FIELDS SEARCHED (IPC) G01N |

The present search report has been drawn up for all claims

| Place of search | Date of completion of the search | Examiner |
|---|---|---|
| Munich | 16 January 2015 | Schwachtgen, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 4415444

- EP 0905229 A2

### Non-patent literature cited in the description

- **OERKE et al.** Crop production and crop protection. Elsevier Science B.V, 1994
- **CONRATH.** *Trends in Plant Science,* 2011, vol. 16 (10), 524-531
- **CONRATH et al.** *Mol. Plant. Microbe Interact,* 2006, vol. 19 (10), 1062-1071
- **HAYES et al.** *J. Leukocyte Biol.,* 1991, vol. 50, 176-181
- **HAYES et al.** *Blood,* 1995, vol. 86, 646-650
- **SIEGRIST et al.** *Physiol. Mol. Plant Pathol,* 1998, vol. 53 (4), 223-238
- **HASE et al.** *Physiological and Molecular Plant Pathology,* 2003, vol. 62, 219-226
- **SIEGRIST et al.** *Physiol. Mol. Plant Pathol.,* 1998, vol. 53 (4), 223-238
- **ANDERLEI ; BUECHS.** *Biochemical Engineering Journal,* 2001, vol. 7, 157-162
- **ANDERLEI et al.** *Biochemical Engineering Journal,* 2004, vol. 17, 187-194
- **ANDERLEI ; BÜCHS.** *Biochemical Engineering Journal,* 2001, vol. 7, 157-162
- **ANDERLE et al.** *Biochemical Engineering Journal,* 2004, vol. 17, 187-194
- **JAMES E. BAILEY ; DAVID F. OLLI.** Biochemical Engineering Fundamentals. 472
- **KNOLL et al.** *Adv. Biochem. Engin/Biotechnol,* 2005, vol. 92, 77-99
- **NÜRNBERGER et al.** *Cell,* 1994, vol. 78, 449-460
- **FELLBRICH et al.** *Plant J.,* 2002, vol. 32 (3), 375-390
- **FELIX et al.** *Plant J.,* 1999, vol. 18 (3), 265-276
- **VILLALBA-MATEOS et al.** *Mol. Plant Microbe Interact.,* 1997, vol. 10, 1045-1053
- **CHALFOUN et al.** *J. Biol. Chem.,* 2013, vol. 288 (20), 14098-14113
- **ROTBLAT et al.** *Plant J.,* 2002, vol. 32 (6), 1049-1055